# FASCICULE DE BREVET EUROPEEN

(11) **EP 0 898 957 B1**
(45) Date de publication et mention de la délivrance du brevet: **26.04.2000**
(21) Numéro de dépôt: 98401641.0
(22) Date de dépôt: 01.07.1998
(51) Int. Cl.: A61K 7/48

(54) **Emulsion eau-dans-huile comprenant de la silice pyrogénée et un alkyléther de polysaccharide**
nasser-in-Öl-Emulsion, die pyrogenes Silikat und einen Polysaccharid-Alkylether enthält
Water in oil emulsion comprising pyrogenic silica and an alkylether of polysaccharide

(30) Priorité: 28.08.1997 FR 9710756
(43) Date de publication de la demande: 03.03.1999
(73) Titulaire: L'OREAL, 75008 Paris (FR)
(72) Inventeur: Sebillotte-Arnaud, Laurence, 94240 L'Hay-Les-Roses (FR)
(74) Mandataire: Tezier Herman, Béatrice

(56) Documents cités:
- EP-A- 0 281 360
- EP-A- 0 708 114
- EP-A- 0 795 321
- EP-A- 0 795 322
- US-A- 5 489 429

## Description

L'invention se rapporte à une émulsion eau-dans-huile et à son utilisation dans les domaines cosmétique, dermatologique, vétérinaire et/ou agroalimentaire. Elle peut se présenter notamment sous forme d'une crème blanche ou colorée, destinée en particulier pour le soin et/ou le maquillage et/ou le démaquillage et/ou la protection solaire de la peau et/ou des muqueuses d'êtres humains ainsi que pour la préparation d'une crème destinée au traitement des maladies de la peau et/ou des muqueuses.

Dans le domaine cosmétique, il est courant d'utiliser des crèmes constituées d'une émulsion eau-dans-huile (E/H) comportant une phase aqueuse dispersée dans une phase grasse liquide généralement appelée phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.

Ces émulsions présentent fréquemment des problèmes de stabilité, rendant leur fabrication difficile. Aussi, différents moyens ont été envisagés pour remédier à cet inconvénient. Un moyen connu consiste à incorporer dans l'émulsion une quantité importante de tensioactif émulsionnant, pouvant aller jusqu'à 10 % en poids par rapport au poids total de l'émulsion. Or, on sait que les émulsionnants utilisés en grande quantité peuvent se montrer irritants vis-à-vis de certains types de peau. Par ailleurs, les crèmes obtenues sont souvent compactes et lourdes. D'autre part, les tensioactifs doivent être choisis en fonction de la polarité des huiles et ne sont donc compatibles qu'avec un nombre limité d'huiles, limitant ainsi la variété des formulations.

Le but de la présente invention est de proposer une émulsion eau-dans-huile ne présentant pas les inconvénients mentionnés ci-dessus et pouvant comprendre une quantité réduite de tensioactif émulsionnant.

Ainsi, la demanderesse a trouvé de manière surprenante qu'il était possible d'obtenir une émulsion eau-dans-huile ayant de bonnes propriétés cosmétiques et une bonne stabilité en utilisant une association particulière d'épaississant de la phase huileuse.

La présente invention a donc pour objet une émulsion eau-dans-huile comportant une phase grasse liquide, caractérisée par le fait que la phase grasse liquide comporte au moins de la silice pyrogénée et au moins un alkyléther de polysaccharide formé de motifs comportant au moins deux cycles osidiques différents, chaque motif comportant au moins un groupe hydroxyle substitué par une chaîne alkyle hydrocarbonée saturée, et de 0 % à 5 % en poids, par rapport au poids total de l'émulsion, d'au moins un tensioactif émulsionnant, la phase grasse liquide contenant au moins un milieu solvant de l'alkyléther de polysaccharide.

Grâce à l'association de silice pyrogénée et de l'alkyléther de polysaccharide, l'émulsion selon l'invention est stable, malgré la faible quantité voire l'absence de tensioactif émulsionnant. La Demanderesse a constaté que la phase aqueuse se disperse parfaitement dans la phase grasse liquide. L'émulsion obtenue est facile à appliquer sur la peau ou les muqueuses et procure une sensation de douceur, de fraîcheur et de confort lors de l'application.

Selon le pourcentage d'épaississant utilisé, on peut obtenir une émulsion de texture plus ou moins fluide. On entend par émulsion de texture fluide, une émulsion s'écoulant sous son propre poids et ayant une viscosité d'environ 2 à 15 poises, soit de 0,2 à 1,5 Pa.s. Une émulsion de texture épaisse selon l'art antérieur a une viscosité d'environ 20 à 80 poises, soit de 2 à 8 Pa.s. L'émulsion selon l'invention présente l'avantage de pouvoir être fluide, crémeuse et confortable tout en présentant une bonne stabilité pendant un mois à température ambiante.

Par comparaison avec l'émulsion selon l'invention, on a constaté qu'une émulsion similaire mais ne comprenant qu'un seul épaississant (silice pyrogénée ou alkyléther de polysaccharide) présente une phase aqueuse dispersée plus grossière et moins stable que l'émulsion de l'invention.

En outre, le demandeur a constaté que l'addition d'alkyléther de polysaccharide permet de diminuer l'aspect rêche d'une émulsion dont la phase huileuse est épaissie uniquement avec de la silice pyrogénée.

Aussi, l'invention a encore pour objet l'utilisation d'un alkyléther de polysaccharide formé de motifs comportant au moins deux cycles osidiques différents, chaque motif comportant au moins un groupe hydroxyle substitué par une chaîne alkyle hydrocarbonée saturée et de silice pyrogénée, pour stabiliser une émulsion eau-dans-huile.

Dans l'épaississant associé à la silice pyrogénée selon l'invention, on entend par « chaîne alkyle hydrocarbonée » une chaîne linéaire ou ramifiée, comportant de 1 à 24, de préférence de 1 à 10, mieux de 1 à 6 et plus spécialement de 1 à 3 atomes de carbone. En particulier, la chaîne alkyle est choisie parmi les chaînes saturées et notamment méthyle, éthyle, éthényle, n-propyle, propényle, isopropyle, n-butyle, isobutyle, tertiobutyle, n-pentyle. Ces alkyléthers peuvent être fabriqués comme décrits dans les documents EP-A-281 360, EP-A-708 114, EP-A-281360.

Selon un mode de réalisation préféré de l'invention, l'alkyléther de polysaccharide a un poids moléculaire moyen en poids supérieur à 100 000, et de préférence supérieur à 200 000. Ce poids moléculaire peut aller jusqu'à 1 million. Cet alkyléther peut comporter de un à six et mieux de deux à quatre groupes hydroxyle par motif, substitués par une chaîne alkyle hydrocarbonée saturée ou non.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide est un alkyléther d'une gomme et plus particulièrement d'une gomme globalement non ionique, c'est-à-dire comportant peu ou pas de groupe ionique. Comme gommes appropriées, on peut citer par exemple la gomme de guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique.

Selon un mode de réalisation préféré de l'invention, l'alkyléther de polysaccharide est un dérivé de gomme de guar. Ainsi, avantageusement l'alkyéther est un galactomannane alkylé de chaîne alkyle en C₁ à C₆ et mieux en C₁ à C₃ et plus particulièrement le guar éthylé ayant un degré de substitution de 2 à 3 et notamment d'environ 2,5 à 2,8, tel que décrit dans les documents RD 95378007 (octobre 1995) et EP-A-708114. Cette gomme est en particulier celles vendues par la société Aqualon sous les noms N-HANCE-AG 200® et N-HANCE AG 50®.

Avantageusement, l'alkyléther de polysaccharide peut être présent en une quantité telle que le rapport (en poids) de la quantité d'huile sur la quantité d'alkyléther de polysaccharide est choisi dans la gamme allant de 5 à 1000.

De façon préférentielle, l'alkyléther de polysaccharide est présent dans la phase grasse liquide de l'émulsion à une concentration en matière active allant de 0,1 à 16 % en poids, par rapport au poids total de l'émulsion et plus préférentiellement de 0,2 à 5 % en poids.

La silice pyrogénée susceptible d'être utilisée dans la composition selon l'invention est de préférence une silice pyrogénée qui peut se présenter sous forme de silice pyrogénée hydrophile ou de silice pyrogénée hydrophobe.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

L'émulsion selon l'invention peut comprendre de la silice pyrogénée en une quantité allant de 0,5 % à 25% en poids, de préférence de 1 à 20% en poids, encore plus préférentiellement de 1 à 10% par rapport au poids total de l'émulsion.

La phase grasse liquide de l'émulsion selon l'invention comprend au moins un milieu solvant de l'alkyléther de polysaccharide qui peut être une huile. En d'autres termes, l'alkyléther de polysaccharide est un épaississant des huiles. Par huile, on entend toute matière grasse liquide à température ambiante.

Parmi les huiles utilisables comme milieu solvant de l'alkyléther de polysaccharide selon l'invention, on peut citer par exemple :
- les huiles d'origine végétale comme les triglycérides liquides, par exemple les huiles de tournesol, de maïs, de soja, de jojoba, de courge, de pépins de raisin, de sésame, de noisette, d'abricot, de macadamia, de ricin, les triglycérides des acides caprylique/caprique comme ceux vendus par la société STEARINERIES DUBOIS ou ceux vendus sous les dénominations MIGLYOL 810, 812 et 818® par la société DYNAMIT NOBEL ;
- les huiles d'origine animale telle que la lanoline ;
- les huiles d'origine minérale ;
- les huiles de synthèse comme les alcools gras tels que l'octyl-2-dodécanol ; les esters et en particulier les esters d'acides gras, et notamment les esters ayant un nombre total d'atomes de carbone choisi entre 12 et 80 et mieux entre 16 et 50 ; les silicones phénylées, et notamment les phényl triméthicones, les diphényl diméthicones, les polyméthylphényl siloxanes.

L'homme du métier sait, par ses connaissances, déterminer par de simples essais de routine les huiles solvantes de l'alkyléther de polysaccharide.

Des huiles complémentaires, non solvant de l'alkyléther de polysaccharide, peuvent en outre être ajoutées dans la phase huileuse de l'émulsion. Comme huile complémentaire, on peut notamment citer les résines et les gommes de silicone liquides à températures ambiantes, les huiles hydrocarbonées partiellement fluorées, les huiles perfluorées, les huiles siliconées exemptes de groupements aromatiques telles que les polysiloxanes linéaires ou ramifiés comme les polydiméthylpolysiloxanes, les polyéthylméthyl polysiloxanes, polyalkylméthylsiloxanes et les polysiloxanes cycliques tels que octaméthylcyclotétrasiloxane, décaméthylcyclopentasiloxane ou leurs mélanges ; les huiles de silicones fluorées ; les polysiloxanes fonctionnalisés par une ou plusieurs fonctions hydroxyles et/ou un ou plusieurs groupements polyéthers tels que les diméthicones copolyols ; les hydrocarbures linéaires ou ramifiés, comme l'huile de vaseline, l'isohexadécane, l'isododécane.

La quantité d'huile que l'on peut introduire dans l'émulsion peut représenter de 20 % à 90 % en poids du poids total de l'émulsion.

Les huiles solvantes de l'alkyléther de polysaccharide peuvent être présentes à raison de 59 à 99,4 % en poids, par rapport au poids total de la phase huileuse de la composition, et mieux de 72 % à 98,5 %. Les huiles complémentaires peuvent être ajoutées dans la composition en une quantité pouvant aller de 0 % à 75 % en poids, par rapport au poids total de la phase huileuse, et de préférence de 0 à 50 % en poids.

On peut en outre ajouter dans la phase huileuse des cires qui sont couramment utilisées dans les compositions cosmétiques. On peut par exemple citer la cire d'abeille, la cire microcristalline, les cires de polyéthylène, les cires de silicone. Les cires peuvent être ajoutées en une teneur allant de 0 à 20 % en poids par rapport au poids de l'émulsion.

Dans l'émulsion selon l'invention, la phase aqueuse peut être présente en une quantité allant de 3 % à 90 % en poids, par rapport au poids total de l'émulsion, de préférence de 3 % à 70 % en poids, et mieux de 10 % à 50 % en poids. La phase aqueuse peut comprendre en outre des gélifiants aqueux comme, par exemple, les acides polyacryliques vendus sous la dénomination "Carbopol®" par la société Goodrich ou bien encore les polymères poly (acide 2-acrylamido 2-méthylpropane sulfonique) vendus sous la dénomination "Hostacerin Amps®" par la société Hoechst.

De façon connue, l'émulsion selon l'invention peut contenir une faible quantité d'un tensioactif émulsionnant d'émulsion E/H. Comme émulsionnants utilisables dans l'invention, on peut citer par exemple les esters d'acide gras et de glucose tels que le dioléate de méthylglucose, les esters d'acide gras et de glycérine tels que l'isostéarate de glycéryle, l'oléate de glycéryle et le ricinoléate de glycéryle, les esters d'acide gras et de sorbitol tels que le tristéarate de sorbitan et le di- ou tri-oléate de sorbitan, les diméthicone copolyols tels que ceux vendus sous la dénomination « DOW CORNING 3225C® » par la société Dow Corning ou sous la dénomination "ABIL EM97®" par la société Goldschmidt, les alkyldiméthiconecopolyol et notamment le céthyl diméthicone copolyol qui est par exemple vendu sous les dénominations « ABIL WE09® » et « ABIL EM90® » par la société Goldschmidt, et plus généralement tout émulsionnant ayant un HLB (balance hydrophile-lipophile) inférieur ou égal à 6. La quantité d'émulsionnant peut représenter de 0,1 à 3 % et de préférence de 0,1 à 2 % du poids total de l'émulsion. Avantageusement, l'émulsion ne comprend pas d'émulsionnant d'émulsion E/H.

L'émulsion selon l'invention est préparée selon les techniques connues de l'homme du métier. En particulier, elle peut être préparée en solubilisant l'alkyléther de polysaccharide dans la phase huileuse chauffée à 80-90 °C, puis en ajoutant au mélange la silice pyrogénée pour obtenir un gel huileux. On ajoute ensuite la phase aqueuse par fraction et après vive agitation, on obtient l'émulsion eau-dans-huile.

Pour une application topique, l'émulsion selon l'invention doit contenir un milieu topiquement acceptable, c'est-à-dire compatible avec la peau, les muqueuses et/ou les fibres kératiniques telles que les cheveux.

De façon connue, l'émulsion de l'invention peut contenir également des adjuvants habituels dans les domaines cosmétique et/ou dermatologique, tels que les actifs, les conservateurs, les antioxydants, les agents complexants, les solvants, les parfums, les charges, les filtres, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse ou dans la phase aqueuse. Leur quantité et leur nature doivent être telles que la stabilité de l'émulsion est conservée.

L'émulsion selon l'invention présente de préférence des globules de phase aqueuse ayant une taille pouvant aller de 10 à 70 µm.

L'émulsion selon l'invention peut également contenir au moins un actif cosmétique et/ou dermatologique pour le soin et/ou le maquillage et/ou le démaquillage et/ou la protection solaire de la peau et/ou des muqueuses.

L'émulsion selon l'invention trouve son application dans un grand nombre de traitements cosmétiques et/ou dermatologiques de la peau, y compris du cuir chevelu, en particulier pour le soin et/ou le maquillage (rouge à lèvres, eye-liner, fond de teint, mascara, anti-cernes, fard à paupières ou à joues) et/ou le démaquillage et/ou la protection solaire de la peau, des cheveux, des cils, des sourcils, des ongles ou des muqueuses. Elle peut aussi être utilisée pour la préparation d'une composition destinée au traitement des maladies de la peau et/ou des muqueuses.

Aussi, la présente invention a encore pour objet l'utilisation cosmétique de l'émulsion telle que définie ci-dessus dans une composition cosmétique pour traiter la peau, les cheveux, les cils, les sourcils, les ongles et/ou les muqueuses.

La présente invention a encore pour objet l'utilisation de l'émulsion telle que définie ci-dessus pour la fabrication d'une composition pharmaceutique et/ou dermatologique destinée au traitement des maladies de la peau et/ou des muqueuses.

La présente invention a aussi pour objet un procédé de traitement non thérapeutique de la peau, des cheveux, des cils, des sourcils, des ongles et/ou des muqueuses, caractérisé par le fait que l'on applique sur la peau, les cheveux, les cils, les sourcils, les ongles et/ou les muqueuses une émulsion ou une composition telles que définies précédemment.

Les exemples ci-après sont donnés à titre d'illustration et sans caractère limitatif.

### Exemples 1 à 4 :

On donne ci après 4 compositions selon l'invention, les compositions 1 et 2 ayant été effectivement réalisés et les compositions 3 et 4 étant des exemples hypothétiques :

| **EXEMPLES** | **Exemple 1** **Conditionneur capillaire** | **Exemple 2** **soin contour des yeux** | **Exemple 3** **Lait corporel** | **Exemple 4** **Crème pour les mains** |
|---|---|---|---|---|
| **Huile de silicone (1)** | 55 | - | 38 | 50 |
| | | | | |
| **triglycérides d'acides caprique/caprylique** | - | 55 | 39 | - |
| **Ethyl guar de degré de substitution d'environ 2,5 (2)** | 0,36 | 0,36 | 0,36 | 0,36 |
| **Silice pyrogénée** | 4,7 | 4 | 2 | 3,5 |
| **hydrophobe (3)** | | | | |
| **Conservateur** | qs | qs | qs | qs |
| | | | | |
| **Glycérine** | - | - | - | 5 |
| **Eau déminéralisée** | QSP 100 | QSP 100 | QSP 100 | QSP 100 |
| **CARACTERISTIQUES** | **Crème épaisse, blanche, très douce et fraîche à l'application.** | **Crème épaisse, blanche, très douce et fraîche à l'application.** | **Lait, blanc, très doux et frais à l'application.** | **Crème épaisse, blanche, très douce et fraîche à l'application.** |

| | | | | |
|---|---|---|---|---|
| (1) vendue sous la dénomination « DOW CORNING 556 FLUID COSMETIQUE ® » par la société Dow Corning | | | | |
| (2) vendu sous la dénomination « N-HANCE AG 200 ® » par la société Aqualon | | | | |
| (3) vendue sous la dénomination « AEROSIL R-972 ® » par la société Degussa | | | | |

Les quantités indiquées sont exprimées en gramme.

Les compositions ont été préparées en solubilisant i'éthylguar dans la phase huileuse chauffée à 80-90 °C puis en ajoutant dans le mélange la silice pyrogénée pour obtenir un gel huileux. Puis on a ajouté petit à petit la phase aqueuse dans le gel huileux et obtenu, après vive agitation, une émulsion eau-dans-huile. On a constaté que les émulsions sont stables au moins un mois à température ambiante.

## Revendications

1. Emulsion eau-dans-huile comportant une phase grasse liquide, caractérisée par le fait que la phase grasse liquide comporte au moins de la silice pyrogénée et au moins un alkyléther de polysaccharide formé de motifs comportant au moins deux cycles osidiques différents, chaque motif comportant au moins un groupe hydroxyle substitué par une chaîne alkyle hydrocarbonée saturée, et de 0 % à 5 % en poids, par rapport au poids total de l'émulsion, d'au moins un tensioactif émulsionnant, la phase grasse liquide contenant au moins un milieu solvant de l'alkyléther de polysaccharide.

2. Emulsion selon la revendication 1, caractérisée par le fait que deux à quatre groupes hydroxyle par motif sont substitués par une chaîne alkyle hydrocarbonée saturée.

3. Emulsion selon l'une quelconque des revendications précédentes, caractérisée par le fait que la chaîne alkyle hydrocarbonée saturée comporte de 1 à 24 atomes de carbone.

4. Emulsion selon l'une quelconque des revendications précédentes, caractérisée par le fait que la chaîne alkyle hydrocarbonée saturée comporte de 1 à 10 atomes de carbone.

5. Emulsion selon l'une quelconque des revendications précédentes, caractérisée par le fait que la chaîne alkyle est choisie dans le groupe formé par les radicaux méthyle, éthyle, n-propyle, isopropyle, n-butyle, isobutyle, tertiobutyle.

6. Emulsion selon l'une quelconque des revendications précédentes, caractérisée par le fait que les cycles osidiques sont choisis dans le groupe formé par le mannose, le galactose, le glucose, le furanose, le rhamnose, l'arabinose.

7. Emulsion selon l'une quelconque des revendications précédentes, caractérisée par le fait que l'alkyléther de polysaccharide est un alkyléther d'une gomme choisie parmi la gomme de guar, la gomme de caroube, la gomme de karaya, la gomme adragante et leurs mélanges.

8. Emulsion selon l'une quelconque des revendications précédentes, caractérisée par le fait que l'alkyéther est un galactomannane alkylé de chaîne alkyle en C₁ à C₆ et mieux en C₁ à C₃.

9. Emulsion selon l'une quelconque des revendications précédentes, caractérisée par le fait que l'alkyléther de polysaccharide est de la gomme de guar à chaîne éthyle avec un degré de substitution de 2 à 3.

10. Emulsion selon l'une quelconque des revendications précédentes, caractérisée par le fait que l'alkyléther de polysaccharide a un poids moléculaire moyen en poids supérieur à 200 000.

11. Emulsion selon l'une quelconque des revendications précédentes, caractérisée par le fait que l'alkyléther de polysaccharide est présent en une quantité telle que le rapport (en poids) de la quantité d'huile sur la quantité d'alkyléther de polysaccharide est choisi dans la gamme allant de 5 à 1000.

12. Emulsion selon l'une quelconque des revendications précédentes, caractérisée par le fait que l'alkyléther de polysaccharide est présent en une quantité allant de 0,1 à 16 % du poids total de la composition et mieux de 0,2 à 5 % du poids total de l'émulsion.

13. Emulsion selon l'une quelconque des revendications précédentes, caractérisée par le fait que la silice pyrogénée est choisi parmi les silices pyrogénées hydrophobes.

14. Emulsion selon l'une quelconque des revendications précédentes, caractérisée par le fait que la silice pyrogénée est présent en une quantité allant de 0,5 % à 25 % en poids, de préférence de 1 % à 20 % en poids, par rapport au poids total de l'émulsion.

15. Emulsion selon l'une quelconque des revendications précédentes, caractérisée par le fait que la phase grasse liquide représente de 20 % à 90 % du poids total de l'émulsion.

16. Emulsion selon l'une quelconque des revendications précédentes, caractérisée par le fait que le milieu solvant de l'alkyléther de polysaccharide est présent en une quantité allant de 59 % à 99,4 % en poids, par rapport au poids total de la phase grasse liquide de la composition, et mieux de 72 % à 98,5 % en poids.

17. Emulsion selon l'une quelconque des revendications précédentes, caractérisée par le fait que la phase grasse liquide comprend au moins une huile complémentaire non solvante de l'alkyléther de polysaccharide.

18. Emulsion selon la revendication 17, caractérisée par le fait que l'huile complémentaire est présente en une quantité allant de 0 % à 75 % en poids, par rapport au poids total de la phase grasse liquide, et de préférence de 0 à 50 % en poids.

19. Emulsion selon l'une quelconque des revendications précédentes, caractérisée en ce qu'elle contient au moins un actif cosmétique et/ou dermatologique pour le soin et/ou le maquillage et/ou le démaquillage et/ou la protection solaire de la peau et/ou des muqueuses.

20. Emulsion selon l'une quelconque des revendications précédentes, caractérisée par le fait qu'elle comprend en outre au moins un ingrédient choisi dans le groupe formé par les conservateurs, les vitamines, les parfums, les antioxydants, les charges, les pigments, les cires, et leurs mélanges.

21. Composition cosmétique ou dermatologique, caractérisée par le fait qu'elle comprend une émulsion selon l'une quelconque des revendications 1 à 20.

22. Utilisation cosmétique de l'émulsion selon l'une quelconque des revendications 1 à 20 dans une composition cosmétique pour traiter la peau, les cheveux, les cils, les sourcils, les ongles et/ou les muqueuses.

23. Utilisation de l'émulsion selon l'une quelconque des revendications 1 à 20 pour la fabrication d'une composition pharmaceutique et/ou dermatologique destinée au traitement des maladies de la peau et/ou des muqueuses.

24. Procédé de traitement non thérapeutique de la peau, des cheveux, des cils, des sourcils, des ongles et/ou des muqueuses, caractérisé par le fait que l'on applique sur la peau, les cheveux, les cils, les sourcils, les ongles et/ou les muqueuses une émulsion selon l'une quelconque des revendications 1 à 20 ou une composition selon la revendication 21.

25. Utilisation d'un alkyléther de polysaccharide, formé de motifs comportant au moins deux cycles osidiques différents, chaque motif comportant au moins un groupe hydroxyle substitué par une chaîne alkyle hydrocarbonée saturée, et d'une silice pyrogénée, pour stabiliser une émulsion eau-dans-huile.

## Patentansprüche

1. Wasser-in-Öl-Emulsion, die eine flüssige Fettphase enthält, dadurch gekennzeichnet, daß
die flüssige Fettphase mindestens pyrogene Kieselsäure und mindestens einen Polysaccharid-Alkylether, der aus Einheiten gebildet ist, die mindestens zwei verschiedene ringförmige Zuckermoleküle enthalten, wobei jede Einheit mindestens eine Hydroxygruppe aufweist, die mit einer gesättigten Kohlenwasserstoffalkylkette substituiert ist, und 0 bis 5 Gew.-%, bezogen auf das Gesamtgewicht der Emulsion, mindestens eines emulgierenden grenzflächenaktiven Stoffs enthält, wobei die flüssige Fettphase mindestens ein Lösungsmittelmedium für den Polysaccharid-Alkylether enthält.

2. Emulsion nach Anspruch 1, dadurch gekennzeichnet, daß 2 bis 4 Hydroxygruppen pro Einheit mit einer gesättigten Kohlenwasserstoffalkylkette substituiert sind.

3. Emulsion nach einem der vorhergehenden Ansprüche, dadurch gekennzeichnet, daß die gesättigte Kohlenwasserstoffalkylkette 1 bis 24 Kohlenstoffatome aufweist.

4. Emulsion nach einem der vorhergehenden Ansprüche, dadurch gekennzeichnet, daß die gesättigte Kohlenwasserstoffalkylkette 2 bis 10 Kohlenstoffatome aufweist.

5. Emulsion nach einem der vorhergehenden Ansprüche, dadurch gekennzeichnet, daß die Alkylkette unter Methyl, Ethyl, n-Propyl, Isopropyl, n-Butyl, Isobutyl, tert.-Butyl ausgewählt ist.

6. Emulsion nach einem der vorhergehenden Ansprüche, dadurch gekennzeichnet, daß die ringförmigen Zuckermoleküle unter Mannose, Galactose, Glucose, Furanose, Rhamnose, Arabinose ausgewählt sind.

7. Emulsion nach einem der vorhergehenden Ansprüche, dadurch gekennzeichnet, daß es sich bei dem Polysaccharid-Alkylether um den Alkylether eines Gummis handelt, das unter Guargummi, Carubingummi, Karayagummi, Adragantgummi und ihren Gemischen ausgewählt ist.

8. Emulsion nach einem der vorhergehenden Ansprüche, dadurch gekennzeichnet, daß es sich bei dem Polysaccharid-Alkylether um ein Galactomannan handelt, das mit C₁-C₆-Alkyl und besser C₁-C₃-Alkyl alkyliert ist

9. Emulsion nach einem der vorhergehenden Ansprüche, dadurch gekennzeichnet, daß es sich bei dem Polysaccharid-Alkylether um ethyliertes Guargummi mit einem Substitutionsgrad von etwa 2 bis 3 handelt.

10. Emulsion nach einem der vorhergehenden Ansprüche, dadurch gekennzeichnet, daß der Polysaccharid-Alkylether ein Gewichtsmittel des Molekulargewichts größer als 200 000 aufweist.

11. Emulsion nach einem der vorhergehenden Ansprüche, dadurch gekennzeichnet, daß der Polysaccharid-Alkylether in einer solchen Menge enthalten ist, daß das Verhältnis (gewichtsbezogen) der Menge an Öl zur Menge an Polysaccharid-Alkylether im Bereich von 5 bis 1000 liegt.

12. Emulsion nach einem der vorhergehenden Ansprüche, dadurch gekennzeichnet, daß der Polysaccharid-Alkylether in einer Menge von 0,1 bis 16 % des Gesamtgewichts der Emulsion und besser 0,2 bis 5 % des Gesamtgewichts der Emulsion enthalten ist.

13. Emulsion nach einem der vorhergehenden Ansprüche, dadurch gekennzeichnet, daß die pyrogene Kieselsäure unter den hydrophoben pyrogenen Kieselsäuren ausgewählt ist.

14. Emulsion nach einem der vorhergehenden Ansprüche, dadurch gekennzeichnet, daß die pyrogene Kieselsäure in einer Menge von 0,5 bis 25 Gew.-%, vorzugsweise 1 bis 20 Gew.-%, bezogen auf das Gesamtgewicht der Emulsion, enthalten ist.

15. Emulsion nach einem der vorhergehenden Ansprüche, dadurch gekennzeichnet, daß der Anteil der flüssigen Fettphase 20 bis 90 % des Gesamtgewichts der Emulsion beträgt.

16. Emulsion nach einem der vorhergehenden Ansprüche, dadurch gekennzeichnet, daß das Lösungsmittelmedium für den Polysaccharid-Alkylether in einem Mengenanteil von 59 bis 99,4 Gew.-%, bezogen auf das Gesamtgewicht der flüssigen Fettphase der Zusammensetzung, und besser in einem Mengenanteil von 72 bis 98,5 Gew.-% enthalten ist.

17. Emulsion nach einem der vorhergehenden Ansprüche, dadurch gekennzeichnet, daß die flüssige Fettphase mindestens ein ergänzendes Öl enthält, in dem sich der Polysaccharid-Alkylether nicht löst.

18. Emulsion nach Anspruch 17, dadurch gekennzeichnet, daß das ergänzende Öl in einem Mengenanteil von 0 bis 75 Gew.-%, bezogen auf das Gesamtgewicht der flüssigen Fettphase, und vorzugsweise 0 bis 50 Gew.-% enthalten ist.

19. Emulsion nach einem der vorhergehenden Ansprüche, dadurch gekennzeichnet, daß sie mindestens einen kosmetischen und/oder dermatologischen Wirkstoff für die Pflege und/oder zum Schminken und/oder Abschminken und/oder für den Sonnenschutz der Haut und/oder der Schleimhäute enthält.

20. Emulsion nach einem der vorhergehenden Ansprüche, dadurch gekennzeichnet, daß sie außerdem mindestens einen Bestandteil enthält, der unter Konservierungsmittel, Vitaminen, Parfums, Antioxidantien, Füllstoffen, Pigmenten, Wachsen und ihre Gemischen ausgewählt ist.

21. Kosmetische oder dermatologische Zusammensetzung, dadurch gekennzeichnet, daß sie eine Emulsion nach einem der Ansprüche 1 bis 20 enthält.

22. Kosmetische Verwendung der Emulsion nach einem der Ansprüche 1 bis 20 in einer kosmetischen Zusammensetzung für die Behandlung der Haut, der Haare, der Wimpern, der Augenbrauen, der Nägel und/oder der Schleimhäute.

23. Verwendung der Emulsion nach einem der Ansprüche 1 bis 20 für die Herstellung einer pharmazeutischen und/oder dermatologischen Zusammensetzung, die für die Behandlungen von Hautkrankheiten und/oder Krankheiten der Schleimhäute vorgesehen ist.

24. Verfahren zur nicht-therapeutischen Behandlung der Haut, der Haare, der Wimpern, der Augenbrauen, der Nägel und/oder der Schleimhäute, dadurch gekennzeichnet, daß auf die Haut, die Haare, die Wimpern, die Augenbrauen, die Nägel und/oder die Schleimhäute eine Emulsion nach einem der Ansprüche 1 bis 20 oder eine Zusammensetzung nach Anspruch 21 aufgetragen wird.

25. Verwendung eines Polysaccharid-Alkylethers, der aus Einheiten gebildet ist, die mindestens zwei verschiedene ringförmige Zuckermoleküle enthalten, wobei jede Einheit mindestens eine Hydroxygruppe aufweist, die mit einr gesättigten Wasserstoffalkylkette substituiert ist, und einer pyrogenen Kieselsäure für die Stabilisierung einer Wasser-in-Öl-Emulsion.

## Claims

1. Water-in-oil emulsion containing a liquid fatty phase, characterized in that the liquid fatty phase contains at least fused silica and at least one polysaccharide alkyl ether formed of units containing at least two different saccharide rings, each unit containing at least one hydroxyl group substituted with a saturated hydrocarbon-based alkyl chain, and from 0% to 5% by weight, relative to the total weight of the emulsion, of at least one emulsifying surfactant, the liquid fatty phase containing at least one medium which is a solvent for the polysaccharide alkyl ether.

2. Emulsion according to Claim 1, characterized in that two to four hydroxyl groups per unit are substituted with a saturated hydrocarbon-based alkyl chain.

3. Emulsion according to either of the preceding claims, characterized in that the saturated hydrocarbon-based alkyl chain contains from 1 to 24 carbon atoms.

4. Emulsion according to any one of the preceding claims, characterized in that the saturated hydrocarbon-based alkyl chain contains from 2 to 10 carbon atoms.

5. Emulsion according to any one of the preceding claims, characterized in that the alkyl chain is chosen from the group formed by the methyl, ethyl, n-propyl, isopropyl, n-butyl, isobutyl and tert-butyl radicals.

6. Emulsion according to any one of the preceding claims, characterized in that the saccharide rings are chosen from the group formed by mannose, galactose, glucose, furanose, rhamnose and arabinose.

7. Emulsion according to any one of the preceding claims, characterized in that the polysaccharide alkyl ether is an alkyl ether of a gum chosen from guar gum, carob gum, karaya gum and gum tragacanth, and mixtures thereof.

8. Emulsion according to any one of the preceding claims, characterized in that the alkyl ether is an alkyl galactomannan with a C₁ to C₆, and better still C₁ to C₃, alkyl chain.

9. Emulsion according to any one of the preceding claims, characterized in that the polysaccharide alkyl ether is guar gum containing an ethyl chain with a degree of substitution of from 2 to 3.

10. Emulsion according to any one of the preceding claims, characterized in that the polysaccharide alkyl ether has a weight-average molecular weight of greater than 200,000.

11. Emulsion according to any one of the preceding claims, characterized in that the polysaccharide alkyl ether is present in an amount such that the ratio (by weight) of the amount of oil to the amount of polysaccharide alkyl ether is chosen in the range from 5 to 1000.

12. Emulsion according to any one of the preceding claims, characterized in that the polysaccharide alkyl ether is present in an amount ranging from 0.1 to 16% of the total weight of the composition, and better still from 0.2 to 5% of the total weight of the emulsion.

13. Emulsion according to any one of the preceding claims, characterized in that the fused silica is chosen from hydrophobic fused silicas.

14. Emulsion according to any one of the preceding claims, characterized in that the fused silica is present in an amount ranging from 0.5% to 25% by weight, preferably from 1% to 20% by weight, relative to. the total weight of the emulsion.

15. Emulsion according to any one of the preceding claims, characterized in that the liquid fatty phase represents from 20% to 90% of the total weight of the emulsion.

16. Emulsion according to any one of the preceding claims, characterized in that the medium which is a solvent for the polysaccharide alkyl ether is present in an amount ranging from 59% to 99.4% by weight, relative to the total weight of the liquid fatty phase of the composition, and better still from 72% to 98.5% by weight.

17. Emulsion according to any one of the preceding claims, characterized in that the liquid fatty phase comprises at least one complementary oil which is not a solvent for the polysaccharide alkyl ether.

18. Emulsion according to Claim 17, characterized in that the complementary oil is present in an amount ranging from 0% to 75% by weight, relative to the total weight of the liquid fatty phase, and preferably from 0 to 50% by weight.

19. Emulsion according to any one of the preceding claims, characterized in that it contains at least one cosmetic and/or dermatological active agent to care for and/or make up and/or remove make-up from and/or provide antisun protection to the skin and/or the mucous membranes.

20. Emulsion according to any one of the preceding claims, characterized in that it also comprises at least one ingredient chosen from the group formed by preserving agents, vitamins, fragrances, antioxidants, fillers, pigments and waxes, and mixtures thereof.

21. Cosmetic or dermatological composition, characterized in that it comprises an emulsion according to any one of Claims 1 to 20.

22. Cosmetic use of the emulsion according to any one of Claims 1 to 20 in a cosmetic composition for treating the skin, the hair, the eyelashes, the eyebrows, the nails and/or mucous membranes.

23. Use of the emulsion according to any one of Claims 1 to 20 for the manufacture of a pharmaceutical and/or dermatological composition intended for treating diseases of the skin and/or mucous membranes.

24. Non-therapeutic treatment process for the skin, the hair, the eyelashes, the eyebrows, the nails and/or mucous membranes, characterized in that an emulsion according to any one of Claims 1 to 20 or a composition according to Claim 21 is applied to the skin, the hair, the eyelashes, the eyebrows, the nails and/or mucous membranes.

25. Use of a polysaccharide alkyl ether formed of units containing at least two different saccharide rings, each unit containing at least one hydroxyl group substituted with a saturated hydrocarbon-based alkyl chain, and a fused silica, to stabilize a water-in-oil emulsion.
